(19) Europäisches Patentamt
European Patent Office
Office européen des brevets

(11) Veröffentlichungsnummer: **0 030 332**
**B1**

(12) # EUROPÄISCHE PATENTSCHRIFT

(45) Veröffentlichungstag der Patentschrift: **27.10.82**

(51) Int. Cl.³: **C 23 F 11/14**, C 10 L 1/22 // C07D233/60, C07D233/94

(21) Anmeldenummer: **80107415.4**

(22) Anmeldetag: **27.11.80**

(54) **Verwendung von 2-Hydroxypropylimidazolen als öllösliche Korrosionsinhibitoren.**

(30) Priorität: **05.12.79 DE 2948884**

(43) Veröffentlichungstag der Anmeldung: **17.06.81 Patentblatt 81/24**

(45) Bekanntmachung des Hinweises auf die Patenterteilung: **27.10.82 Patentblatt 82/43**

(84) Benannte Vertragsstaaten: **BE DE FR GB IT NL**

(56) Entgegenhaltungen:
**DE-A-1 954 706**
**US-A-3 280 139**

(73) Patentinhaber: **BASF Aktiengesellschaft, Carl-Bosch-Strasse 38, D-6700 Ludwigshafen (DE)**

(72) Erfinder: **Vogel, Hans-Henning, Dr., Hans-Purrmann-Strasse 7C, D-6710 Frankenthal (DE)**
Erfinder: **Strickler, Rainer, Dr., Schroederstrasse 14, D-6900 Heidelberg (DE)**
Erfinder: **Oppenlaender, Knut, Dr., Otto-Dill-Strasse 23, D-6700 Ludwigshafen (DE)**
Erfinder: **Baur, Richard, Dr., Schillerstrasse 10, D-6701 Dannstadt-Schauernheim (DE)**

**EP 0 030 332 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verwendung von 2-Hydroxypropylimidazolen als öllösliche Korrosionsinhibitoren

Die Erfindung betrifft die Verwendung von substituierten 2-Hydroxypropylimidazonen als Korrosionsinhibitoren in Kohlenwasserstoffen.

Auf zahlreichen Anwendungsgebieten, bei denen Metalle mit Wasser aber auch mit Öl-Wasser-Zweiphasensystemen, wie z. B. wäßrigen Emulsionen oder anderen Stoffsystemen, die Wasser enthalten, in Kontakt stehen, besteht die Gefahr der Korrosion. Es ist bekannt, daß auch durch Flüssigkeiten, die nur geringe Mengen Wasser enthalten, wie Otto- und Dieselkraftstoffen, Korrosion verursacht werden kann: Kondenswasser sammelt sich in Tropfenform am Boden von Kraftstofftanks oder in Rohrleitungen und wirkt zusammen mit aus der Luft aufgenommenen Sauerstoff korrosiv, was zu Schäden an Blech- und Bleischicht von Tanks, aber auch am ganzen Kraftstoffleitungssystem von Motoren führen kann. In der Regel ist man bemüht, die Korrosionswirkung einer Flüssigkeit gegenüber Maschinen- und Apparateteilen, Behältern und Rohrwandungen und sonstigen metallischen Konstruktionselementen durch Korrosionsinhibitoren zu verhindern oder aufzuheben. Zur Verhinderung der Korrosion des Eisens werden normalerweise in nicht-wäßrigen Flüssigkeiten langkettige, hochmolekulare organische Säuren, Ester oder Amine mit oberflächenaktiven Eigenschaften eingesetzt. Als Korrosionsschutz-Additive für Buntmetalle sind Benzo- und Tolyltriazol aus DE-B-2 265 088 bekannt.

Benzo- und Tolyltriazol sind zwar universell einsetzbare Korrosionsinhibitoren für Buntmetalle, aber ihre aufwendige Synthese bringt hohe Kosten mit sich, und durch ihren relativ polaren Charakter ist ihre Löslichkeit in Medien, die im wesentlichen nur aliphatische Kohlenwasserstoffe enthalten, begrenzt. Das Ziel der Erfindung bestand daher in der Bereitstellung öllöslicher Korrosionsinhibitoren, die ein möglichst breites Wirkungs- und Anwendungsspektrum besitzen und die oben genannten Nachteile nicht aufweisen.

Überraschenderweise wurde nun gefunden, daß öllösliche, 2-Hydroxypropylimidazolderivate der Formel I sehr gute Korrosionsinhibitoren sind,

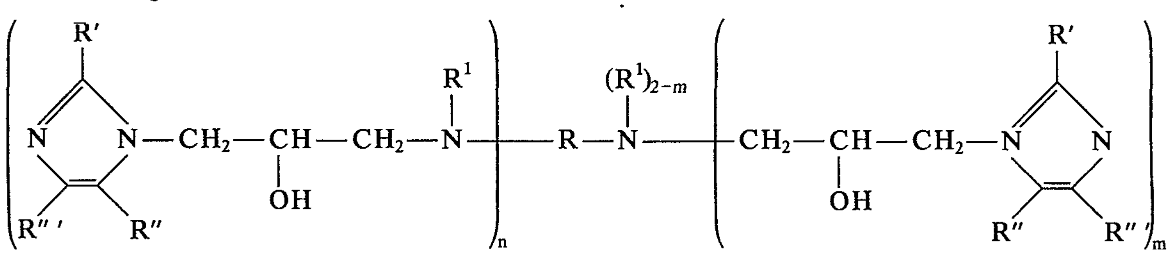

(I)

in der m die Zahlen 1 oder 2 und n 0 oder 1 bedeutet, $R^1$ einen aliphatischen oder cycloaliphatischen Rest mit 6 bis 21 Kohlenstoffatomen, R im Falle von n gleich 0 einen aliphatischen, cycloaliphatischen, aromatischen oder araliphatischen Rest mit 6 bis 21 Kohlenstoffatomen bedeutet und vorzugsweise mit $R^1$ gleich ist oder

wenn n die Zahl 1 bedeutet einen zweiwertigen aliphatischen oder aromatischen Rest mit 2 bis 15 Kohlenstoffatomen oder den Rest der Formel

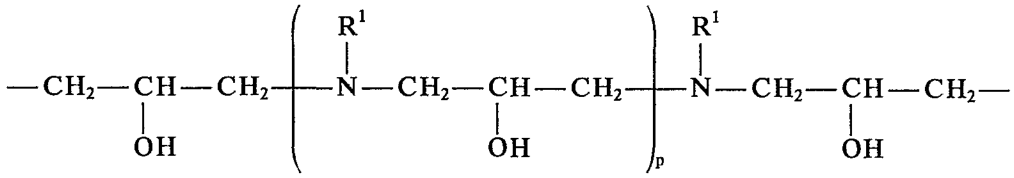

in der p für die Zahl 0 oder 2 steht, bedeutet und R′, R″, R‴ für Wasserstoffatome oder Alkylreste mit 1 bis 4 Kohlenstoffatomen stehen und R″ oder R‴ ferner eine Nitrogruppe bedeuten können.

Diese Hydroxypropylimidazole erhält man z. B. durch Umsetzung von Imidazolen mit freier N—H-Gruppe, mit Mono- ober Bis-2,3-Epoxypropylaminen (III ) bzw. 3-Halogen-2-hydroxy-propylaminen (IV) (gegebenenfalls unter Zusatz von Aminen der Formel $r^1-NH_2$) in Substanz oder in üblichen Lösungsmitteln wie Methanol, Äthanol, Isopropanol, Isobutanol, Methylglykol, Aceton, Methyläthylketon, Tetrahydrofuran, Dioxan, Dimethylformamid, N-Methylpyrrolidon, Essigsäureäthylester, Toluol, Benzol oder Xylol bei Temperaturen zwischen 0 und 160° C, bevorzugt zwischen 80 und 120° C.

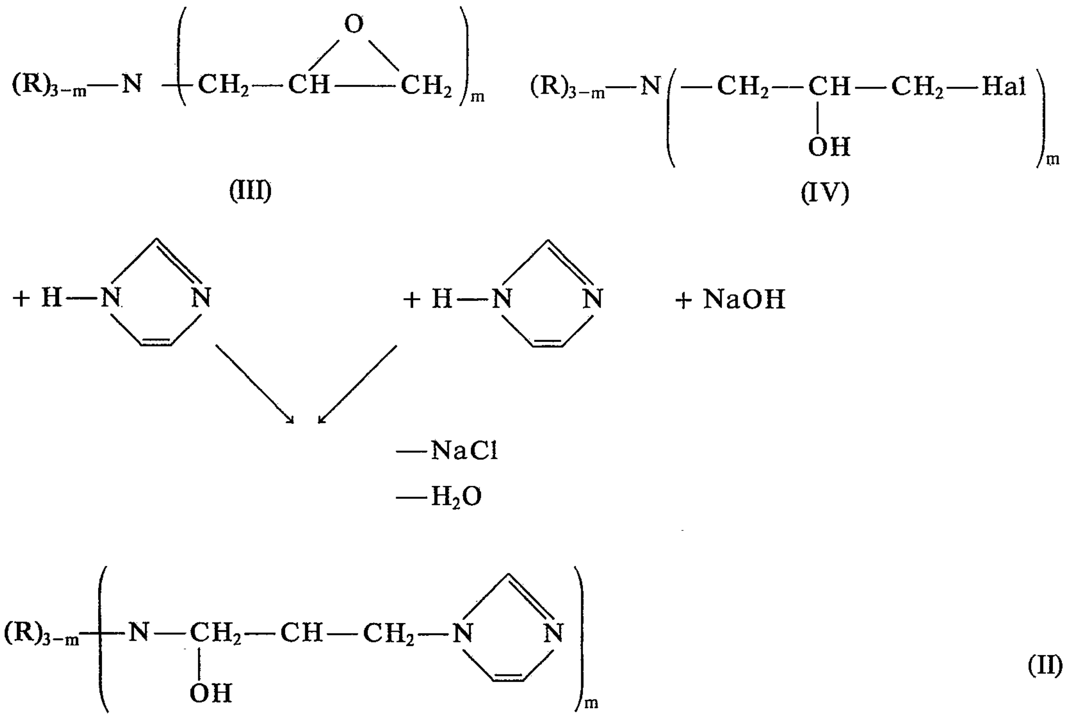


(III) (IV) (II)

Die Reaktion kann leicht durch Titration der Epoxidgruppen bzw. des freien Chlorids kontrolliert werden.

3-Halogen-2-hydroxypropylamine (IV) sind nach literaturbekannten Methoden durch Umsetzung von Aminen mit Epihalogenhydrin zugänglich; die alkalische Zyklisierung dieser Halogenhydrine führt im nächsten Schritt zu 2,3-Epoxypropylamin (III) (GB 772 830, J. Org. Chem., 28, 2283 (1963)).

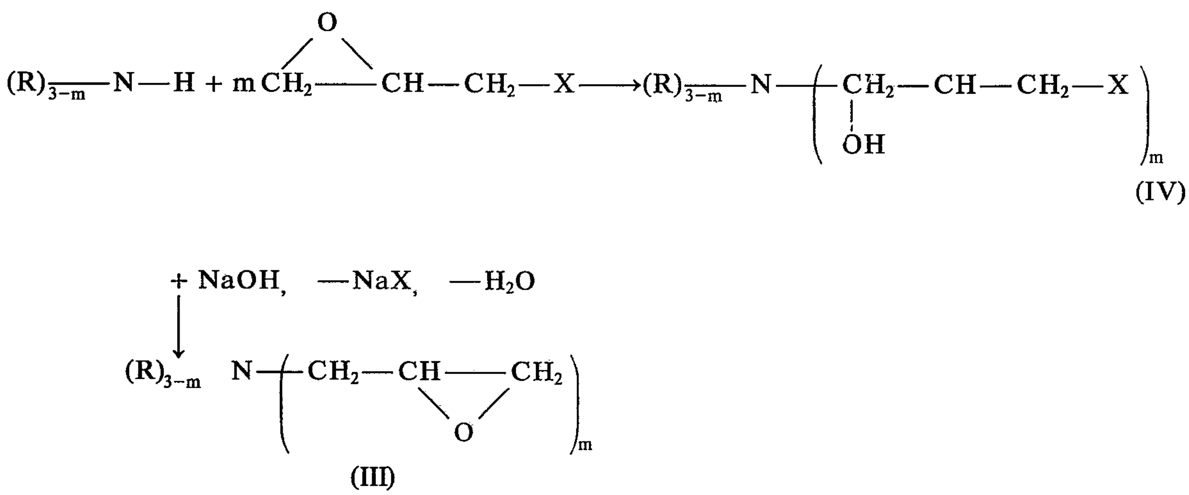


(IV) (III)

Verbindungen der Formel I, in der R den Rest

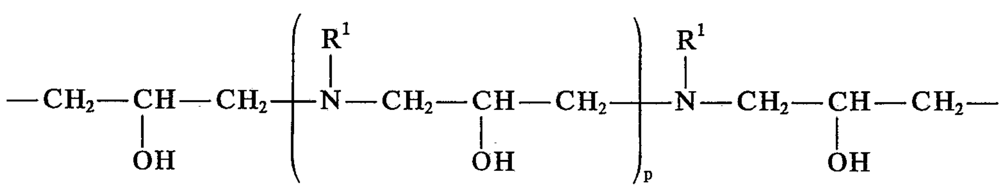

bedeutet werden z. B. erhalten, wenn man 2 bis 3 Mol der Bis-2,3-Epoxypropylamine der Formel V

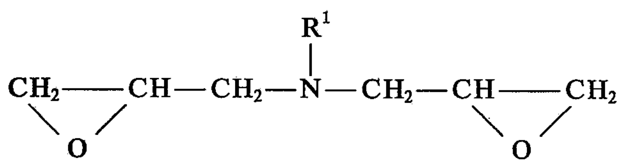

mit 1 bis 2 Mol Aminen der Formel $R^1-NH_2$ und 2 Mol Imidazol umsetzt.

Unter den Aminen, die mit Epihalogenhydrin umgesetzt werden können, seien genannt: Alkylamine wie n-Butylamin, Cyclohexylamin, Dodecylamin, Isotridecylamin, Oleylamin, Alkoxypropylamine wie 2-Äthylhexoxypropylamin, Arylamine wie Anilin, das gegebenenfalls durch nichtionogene Gruppen substituiert sein kann und Polyamine beispielsweise aliphatische oder aromatische Di- oder Polyamine wie 4,4'-Diaminodiphenylmethan, 4,7-Dioxadodecan-1,12-diamin usw.

In den bevorzugten Hydroxypropylimidazolderivaten der Formel I sind R', R'', R''' Wasserstoffatome oder Methylgruppen. Insbesondere sind Derivate des Imidazols selbst und des 2-Methylimidazols zu nennen.

Eine besonders bevorzugte Untergruppe sind die Verbindungen der Formel II

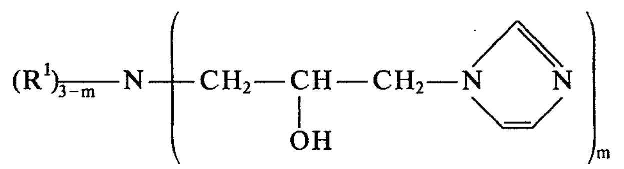

(II)

in der m die oben angegebene Bedeutung hat und $R^1$ eine Alkyl- oder Cycloalkylrest mit 6 bis 18 Kohlenstoffatomen, vor allem einen Isotridecylrest bedeutet.

Die erfindungsgemäßen Hydroxypropylimidazole sind für eine Vielzahl von Anwendungszwecken als Korrosionsinhibitoren, insbesondere in Kohlenwasserstoffen, wirksam.

Die Anwendungszwecke schließen technische Wärmeübertragungssysteme, Pumpenrohre und andere Metalleinrichtungen für Öl-Förderanlagen und Pipelines, insbesondere Kraftstoffleitungen von Benzin- und Dieselmotoren sowie alle Motorteile wie Vergaser, Einspritzpumpe, Kolben usw., aber auch Tanks zur Lagerung und zum Transport von Kraftstoffen mit ein. Als besonders korrosionsgefährdet gelten dabei die Vergaserbauteile (bestehend aus Zinkspritzguß = 95% Zink, 4% Aluminium und 1% Kupfer) sowie die verbleiten Teile von Kraftstofftanks.

Der Korrosionsinhibitor kann dabei direkt in Konzentrationen von 0,1 bis 1000 ppm, bevorzugt von 1 bis 500 ppm dem Kraftstoff zugesetzt werden. Weiterhin möglich ist auch die Zugabe des Inhibitors im Rahmen eines handelsüblichen Kraftstoff-Additiv-Gemisches, bestehend aus einem Ventil- und Vergaserreiniger, einem Oxidationsinhibitor, einem filmbildenden Korrosionsschutzadditiv (zur Verhinderung der Korrosion des Eisens) usw.

Das Korrosionsverhalten von Wasser enthaltenden Kraftstoffen wird üblicherweise an Metallcoupons in Kraftstoff-Wasser-Gemischen geprüft. Die Wirkung der Produkte ist den folgenden Beispielen zu entnehmen.

## Beispiel

### N-Cyclohexyl-N,N-bis-(3-imidazolyl-2-hydroxypropyl)amin

a) 68,1 g Imidazol werden in einer 500 ml-Rührapparatur mit Rückflußkühler, Tropftrichter, Rührer und Kontaktthermometer unter Stickstoff aufgeschmolzen. Bei 100°C tropft man innerhalb einer Stunde 115,0 g N-Cyclohexyl-N,N-bis-2,3-epoxypropylamin (91,7%iges Produkt) zu. Nach einer weiteren Stunde ist kein Epoxid mehr im Reaktionsgemisch nachweisbar. Man läßt abkühlen und erhält 173,0 g eines pastösen Produktes.

| | | |
|---|---|---|
| $N_{basisch}$ | 8,2 mval/g | (Theorie 8,6 mval/g) |
| $N_{Gesamt}$ | 14,2 mval/g | (Theorie 14,4 mval/g) |
| OH-Zahl | 314,4 | (Theorie 322,8) |

### b) Korrosionstest

Eine 250 ml-Glasflasche wird mit 100 ml Superbenzin (Erdölraffinerie Mannheim) gefüllt. Das Produkt wird in Mengen zwischen 10 und 1000 ppm dosiert. Dann werden 4 ml destilliertes Wasser zugegeben. Testcoupons der Größe 50 mm x 20 mm x 2 mm werden geschmirgelt (Körnung 20), mit Toluol entfettet und gewogen. Die Testflaschen schüttelt man eine Minute kräftig, damit das Wasser im Benzin verteilt wird. Die Metallcoupons werden eingelegt und 14 Tage bei 20 bis 25°C gelagert. Dann werden die Coupons mit 15%iger Salzsäure, die noch 1% Propargylalkohol als Beizinhibitor enthält, gereinigt, entfettet und getrocknet. Die Bestimmung des Gewichtsverlustes erfolgt durch Wiegen (Angaben in ‰ Gewichtsverlust). In der Regel werden Mehrfachbestimmungen durchgeführt.

| Dosierung | Metall<br>Blei |
|---|---|
| Ohne Zusatz | 1.84‰ |
| Verb. des Beispiels 1 | |
| 10 ppm | 1.48‰ |
| 50 ppm | 1.37‰ |
| Additiv-Gemisch*) | |
| 300 ppm | 0.45‰ |
| 500 ppm | 0.48‰ |
| Additiv-Gemisch mit 1 Gew.-% bezogen auf das Gemisch der Verb. d. Beispiels 1 | |
| 300 ppm | 0.40‰ |
| 500 ppm | 0.14‰ |

*) Additiv-Gemisch eines handelsüblichen Ventil- und Vergaserreinigers ohne Benzotriazol als Korrosionsinhibitor.

## Beispiel 2

### N-2-Äthylhexyl-N,N-bis-(3-imidazolyl-2-hydroxypropyl)amin

a) 68,1 g Imidazol (1 Mol) werden in einer 500 ml-Rührapparatur mit Tropftrichter, Rückflußkühler, Rührer und Kontaktthermometer in 200 ml Äthylmethylketon gelöst. Das Reaktionsgemisch wird dann unter Stickstoff zum Sieden erhitzt (80° C) und man tropft 126,3 g 95,4%iges 2-Äthylhexyl-bis-2,3-epoxypropylamin (0,5 Mol) innerhalb einer Stunde zu. Schließlich destilliert man das Lösungsmittel ab; es ist dann kein Epoxid mehr im Ansatz nachweisbar. Man erhält so 194,4 g des pastösen N-2-Äthylhexyl-N,N-bis-(3-imidazolyl-2-hydroxypropyl)amins.

| | | |
|---|---|---|
| $N_{basisch}$ | 7,7 mval/g | (Theorie 8,0 mval/g) |
| $N_{Gesamt}$ | 13,2 mval/g | (Theorie 13,3 mval/g) |
| OH-Zahl | 293,1 | (Theorie 297,0) |

b) Korrosionstest 2, analog zu Beispiel 1b)

Angaben in ‰ Gewichtsverlust

| Dosierung | Metall | |
|---|---|---|
| | Blei | Zinkspritzguß 95% Zn, 4% Al, 1% Cu |
| Ohne Zusatz | 1.53 | 3,72 |
| Zusatz gem. Beispiel 2 | | |
| 10 ppm | 0.80 | 1,50 |
| 20 ppm | 0.33 | 1,18 |
| 30 ppm | 0.34 | 1,07 |
| 50 ppm | 0.48 | 1,40 |
| Additivgemisch ohne Zusatz eines Korrosionsinhibitors | | |
| 300 ppm | 0.45 | 0,05 |
| 500 ppm | 0.48 | 0,06 |
| Additivgemisch + 1% der Verb. gem. Beispiel 2 | | |
| 300 ppm | 0.34 | 0,04 |
| 500 ppm | 0.29 | 0,04 |

Beispiel 3

N-Isotridecyl-N,N-bis-(3-imidazolyl-2-hydroxypropylamin

a) 68,1 g Imidazol (1 Mol) werden in einer 1 l-Rührapparatur mit Rückflußkühler, Rührer, Tropftrichter und Kontaktthermometer bei 100° C in 250 ml Toluol vorgelegt. Dann tropft man im Laufe einer Stunde 173,9 g 89,4%iges Tridecyl-bis-2,3-epoxypropylamin (0,5 Mol) zu und hält noch eine Stunde bei 100° C. Dann destilliert man im Wasserstrahlvakuum das Lösungsmittel ab. Der Rückstand enthält keine Epoxidgruppen mehr. Man erhält so 239,6 g N-Isotridecyl-N,N-bis-(3-imidazolyl-2-hydroxypropyl)amin.

| | | |
|---|---|---|
| $N_{Gesamt}$ | 11,2 mval/g | (Theorie 11,2 mval/g) |
| $N_{basisch}$ | 6,2 mval/g | (Theorie 6,7 mval/g) |
| OH-Zahl | 243,5 | (Theorie 250,5) |

IR-Spektrum: Banden bei 3500—3100, 1520, 1465, 1380, 1235, 1070 und 750 $cm^{-1}$.

b) Korrosionstest

Angaben in ‰ Gewichtsverlust

| Dosierung | Metall | | |
|---|---|---|---|
| | Blei | Zinkspritzguß | Stahl |
| Ohne Zusatz | 1.83 | 3.70 | 1.74 |
| Verbindung gemäß Beispiel 3a | | | |
| 10 ppm | 0.40 | 1.22 | |
| 50 ppm | 0.55 | 1.20 | |
| 100 ppm | 0.45 | 1.01 | |
| 500 ppm | | | 0.09 |
| 1000 ppm | | | 0.08 |
| Additivgemisch ohne Zusatz eines Korrosionsinhibit. | | | |
| 100 ppm | 1.91 | | |
| 300 ppm | 0.68 | 1.55 | |
| 500 ppm | 0.56 | 1.13 | |
| Additivgemisch + 1 Gew.-% der Verbindung gem. Beispiel 3 | | | |
| 100 ppm | 1.77 | | |
| 300 ppm | 0.27 | 1.40 | |
| 500 ppm | 0.36 | 1.28 | |

c) 185 g Epichlorhydrin (2 Mol) und 15 ml Wasser werden bei 30 bis 35°C in einer Rührapparatur mit Rückflußkühler, Tropftrichter, Rührer und Thermometer vorgelegt. Bei dieser Temperatur tropft man unter leichtem Kühlen 199 g Isotridecylamin (1 Mol) zu und rührt nach bis kein freies Epichlorhydrin mehr titrierbar ist (8 Stunden).

Dann läßt man 136,2 g Imidazol (2 Mol) in 400 ml Isobutanol zufließen, bringt auf 80°C und tropft dabei langsam 280 g 50%ige Kalilauge (2,5 Mol) zu. Nach 4 Stunden zeigt eine Chloridbestimmung, daß das Chlorhydrin vollständig umgesetzt wurde. Man verdünnt nun mit ca. 300 ml Wasser, trennt die Phasen und wäscht nochmals mit insgesamt 1 l Wasser nach. Die organische Phase wird am Rotationsverdampfer eingeengt. Es verbleiben 305 g eines viskosen Produktes, dessen IR-Spektrum mit dem des Produkts aus Beispiel 3a identisch ist.

d) Korrosionstest

| Dosierung | Metall | |
|---|---|---|
| | Blei | Zinkspritzguß |
| Ohne Zusatz | 1.83 | 3.70 |
| Verbindung gem. Beispiel 3b | | |
| 10 ppm | 0.60 | 1.61 |
| 50 ppm | 0.52 | 1.78 |
| Additivgemisch ohne Zusatz eines Korrosionsinhibit. | | |
| 300 ppm | 0.68 | 1.55 |
| 500 ppm | 0.56 | 1.13 |
| Additivgemisch + 1 Gew.-% der Verb. gem. Beispiel 3b | | |
| 300 ppm | 0.23 | 1.38 |
| 500 ppm | 0.24 | 0.99 |

## Beispiel 4

### Di-2-äthylhexyl-(3-imidazolyl-2-hydroxypropyl)amin

a) In einem 1 l-Rührkolben mit Tropftrichter, Rührer, Thermometer und Rückflußkühler läßt man bei 35° C 241,5 g Di-2-äthylhexylamin (1 Mol) zu 92,5 g Epichlorhydrin (1 Mol) und 10 ml Wasser zufließen und hält noch 22 Stunden bei dieser Temperatur. Zu diesem Zeitpunkt liegt im Reaktionsgemisch kein freies Epichlorhydrin mehr vor. Dann läßt man 68,1 g Imidazol (1 Mol) in 400 ml Isobutanol zufließen, erhitzt auf 100° C und troft im Laufe von zwei Stunden 140 g 50%ige Kalilauge (1,25 Mol) zu.

Nach 3 Stunden sind 1,13 mVal Chlorid/g im Ansatz nachweisbar. Man läßt abkühlen und wäscht mehrmals mit insgesamt 1,5 l Wasser aus. Es verbleiben nach Einengen der organischen Phase im Vakuum 273 g des Di-2-äthylhexyl-(3-imidazolyl-2-hydroxypropylamin zurück.

| | | |
|---|---|---|
| $N_{Gesamt}$ | 7,9 mval/g | (Theorie 8,2 mval/g) |
| $N_{basisch}$ | 5,0 mval/g | (Theorie 5,5 mval/g) |
| OH-Zahl | 158,9 | (Theorie 153,4) |

| Dosierung | Metall | |
|---|---|---|
| | Blei | Zinkspritzguß |
| Ohne Zusatz | 1.84 | 3.49 |
| Verbindung gem. Beispiel 4 | | |
| 50 ppm | 1.30 | 2.28 |
| Additivgemisch ohne Zusatz eines Korrosionsinhibitors | | |
| 500 ppm | 0.48 | 0.06 |
| Additivgemisch + 1% der Verb. gemäß Beispiel 4 | | |
| 500 ppm | 0.29 | 0.04 |

Beispiel 5

Oligo-N-tricecyl-imidazolyl-hydroxypropylamin

a)

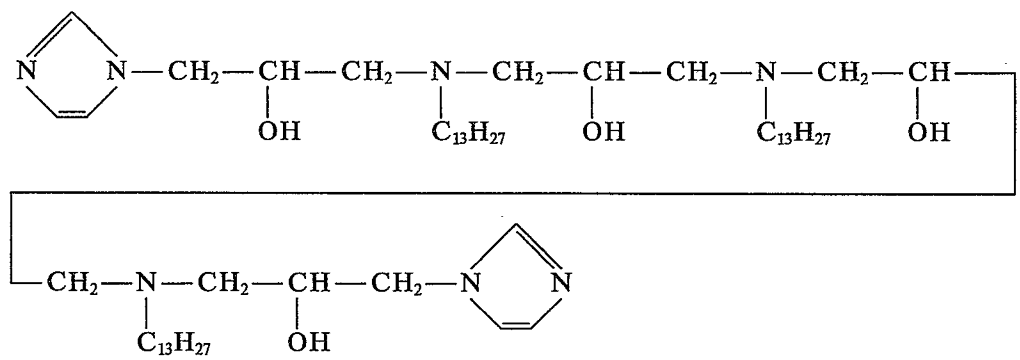

68,1 g Imidazol (1 Mol) werden in einer 1-l-Rührapparatur mit Rückflußkühler, Rührer, Tropftrichter und Kontaktthermometer bei 100° C in 250 ml Isobutanol vorgelegt. Man tropft zunächst im Laufe einer Stunde 347,8 g 89,4 proz. Tridecylbis-2,3-epoxipropylamin (1 Mol) und dann 99,5 g Isotridecylamin (0,5 Mol) zu und hält noch eine Stunde eine Temperatur von 100° C aufrecht. Dann destilliert man im Wasserstrahlvakuum das Lösungsmittel ab. Der Rückstand enthält keine Epoxidgruppen mehr. Man erhält so 509 g eines Oligotridecylimidazolylhydroxypropylamins.

| | | |
|---|---|---|
| $N_{Ges}$ | 6,9 mval/g | (Theorie: 7,3 mval/g) |
| $N_{Basisch}$ | 4,9 mval/g | (Theorie: 5,2 mval/g) |

b) Korrosionstest: Verbindung gemäß Beispiel 5a

| Dosierung | Metall | |
|---|---|---|
| | Blei | Zinkspritzguß |
| 0 | 0,82‰ | 3,57‰ |
| 10 ppm | 0,12‰ | 2,62‰ |
| 20 ppm | 0,12‰ | 0,46‰ |
| 50 ppm | 0,20‰ | 0,37‰ |

Beispiele 6 bis 12

Wie in Beispiel 2 beschrieben wurden die folgenden Verbindungen hergestellt und getestet:

Tabelle

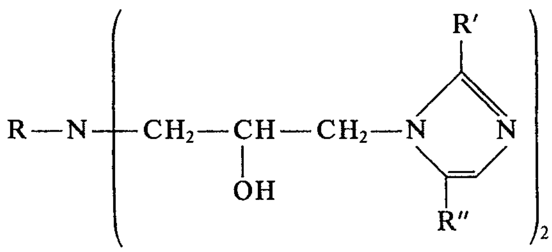

| | Verbindung | | | | |
|---|---|---|---|---|---|
| | $N_{Gesamt}$ | OHZ | Relativer Abtrag in ‰ | | |
| | Dosierung je 50 ppm | | | | |
| | (Theorie) in mval/g | (Theorie) | Blei | Zinkspritzguß | Kupfer |
| Blindprobe | | | 0,82 | 3,51 | 0,04 |
| Beispiel 6 | 11,3 | 262,5 | | | |
| R = n-$C_{12}H_{25}$ | | | 0,33 | 0,83 | |
| R' = R'' = H | (11,5) | (258,6) | | | |
| Beispiel 7 | 10,2 | 228,5 | | | |
| R = Oleyl | | | 0,30 | 0,62 | |
| R' = R'' = H | (9,7) | (217,5) | | | |
| Beispiel 8 | 9,1 | 218,8 | | | |
| R = $C_3H_6OC_{13}H_{27}$ | | | 0,04 | 0,084 | |
| R' = R'' = H | (9,9) | (221,8) | | | |
| Beispiel 9 | 10,6 | 239,3 | | | |
| R = $C_3H_6OC_8H_{17}$ | | | 0,22 | 0,76 | |
| R' = R'' = H | (11,5) | (257,5) | | | |
| Beispiel 10 | 10,2 | 227,5 | | | |
| R = $C_{13}H_{27}$ | | | 0,30 | 0,49 | 0,01 |
| R' = $CH_3$, R'' = H | (10,5) | (235,8) | | | |
| Beispiel 11 | 8,8 | 218,0 | | | |
| R = $C_{13}H_{27}$ | | | 0,25 | 0,41 | 0 |
| R' = $C_3H_7$, R'' = H | (9,4) | (210,9) | | | |

Fortsetzung

| | Verbindung $N_{Gesamt}$ (Theorie) in mval/g | OHZ (Theorie) | Relativer Abtrag in ‰ Blei | Zinkspritzguß | Kupfer |
|---|---|---|---|---|---|
| | Dosierung je 50 ppm | | | | |
| Beispiel 12 | 11,9 | 216,5 | | | |
| $R = C_{13}H_{27}$ | | | 0,12 | 0,40 | 0,01 |
| $R' = H$, $R'' = NO_2$ | (13,0) | (208,6) | | | |

Test der Kupferkorrosion bei Zusatz von 2-Hydroxypropylimidazolen

Testmethode:

In zylindrischen Probegläsern (wie in DIN 51 538, Maße: Höhe 180 mm, Durchmesser 40 mm) werden Kupferstreifen geprüft (Maße: 100 × 8 × 2 mm). Die Streifen werden mit 150er Schmirgelleinen geschmirgelt, mit Toluol entfettet und gewogen. Prüflösung: Solventraffinat, Viskosität 6 $mm^2/s$ bei 100° C, enthält 50 gew.-ppm Schwefel gelöst sowie den zu untersuchenden Inhibitor.

Die Proben werden 3 bzw. 6 Stunden im Trockenschrank stehengelassen und nach Abkühlen auf Raumtemperatur optisch bewertet.

Bewertungsskala:

Rating 1 — unverändert, kein Angriff
Rating 2 — rötliche Anlauffarben
Rating 3 — bis zu 50% der Metalloberfläche schwarz
Rating 4 — bis zu 80% der Metalloberfläche schwarz
Rating 5 — über 80% der Metalloberfläche schwarz

Tabelle

Kupferkorrosion in Schmieröl nach ASTMD 130 in Gegenwart von Schwefel

| Produkt | Rating nach 3 Stunden bei Dosierung ppm | | Rating nach 6 Stunden bei Dosierung ppm | |
|---|---|---|---|---|
| | 100 | 500 | 100 | 500 |
| Blindwert | | 4–5 | | 5 |
| Beispiel 3 | 2 | 1 | 1 | 1 |
| Beispiel 4 | 2 | 1 | 2 | 1 |
| Beispiel 5 | 1 | 1 | 1 | 1 |
| Beispiel 10 | 1 | 1 | 1 | 1 |
| Beispiel 11 | 1 | 1 | 1 | 1 |
| Beispiel 12 | 1 | 1 | 2 | 1 |
| Benzotriazol | 2 | 2 | | |

**Patentansprüche**

1. Verwendung von 2-Hydroxypropylimidazolderivaten der Formel I

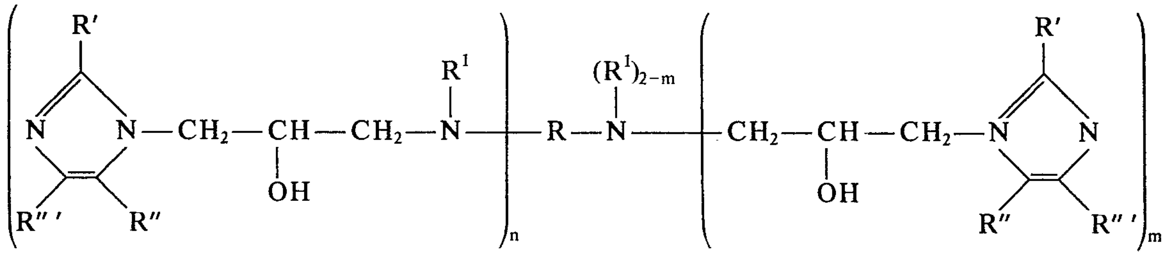

(I)

in der m die Zahlen 1 oder 2 und n 0 oder 1 bedeutet, $R^1$ einen aliphatischen oder cycloaliphatischen Rest mit 6 bis 21 Kohlenstoffatomen, R im Falle von n gleich 0 einen aliphatischen, cycloaliphatischen, aromatischen oder araliphatischen Rest mit 6 bis 21 Kohlenstoffatomen bedeutet und vorzugsweise mit $R^1$ gleich ist oder
wenn n die Zahl 1 bedeutet einen zweiwertigen aliphatischen oder aromatischen Rest mit 2 bis 15 Kohlenstoffatomen oder den Rest der Formel

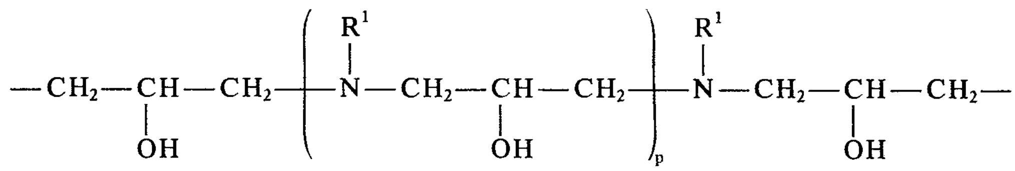

in der p für die Zahlen 0 oder 2 steht, bedeutet und R', R'', R''' für Wasserstoffatome oder Alkylreste mit 1 bis 4 Kohlenstoffatomen stehen und R'' oder R''' ferner eine Nitrogruppe bedeuten können, als Korrosionsinhibitoren in Kohlenwasserstoffen.

2. Verwendung als Korrosionsinhibitoren in Kohlenwasserstoffen von 2-Hydroxypropylimidazolderivaten der Formel

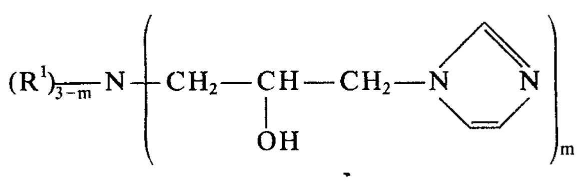

(II)

in der $R^1$ ein Alkyl- oder Cycloalkylrest mit 6 bis 18 Kohlenstoffatomen und m die Zahlen 1 oder 2 bedeutet.

**Claims**

1. Use of 2-hydroxypropylimidazole derivatives of the formula I

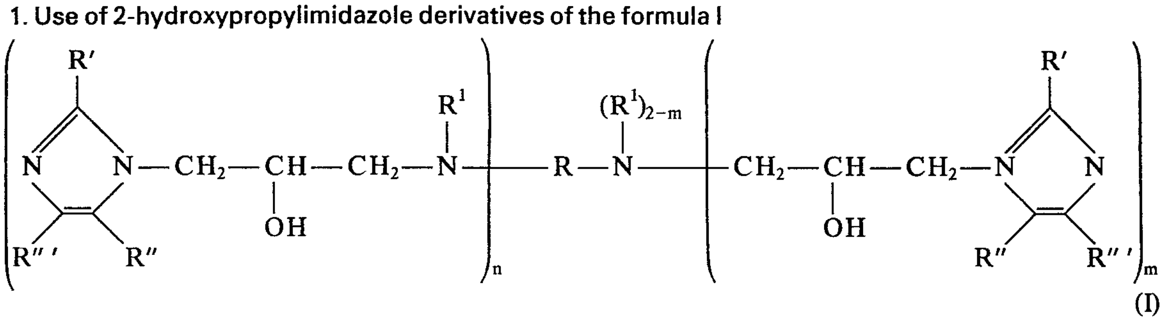

(I)

where m is 1 or 2 and n is 0 or 1, $R^1$ is an aliphatic or cycloaliphatic radical of 6 to 21 carbon atoms, R, if n is 0, is an aliphatic, cycloaliphatic, aromatic or araliphatic radical of 6 to 21 carbon atoms and is preferably identical with $R^1$, or R, if n is 1, is a divalent aliphatic or aromatic radical of 2 to 15 carbon atoms or is the radical of the formula

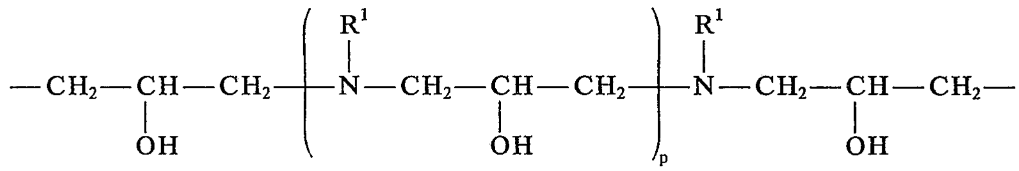

p being 0 or 2, and R′, R″ and R‴ are hydrogen atoms or alkyl groups of 1 to 4 carbon atoms, and R″ or R‴ may also be nitro, als corrosion inhibitors in hydrocarbons.

2. Use of 2-hydroxypropylimidazole derivatives of the formula

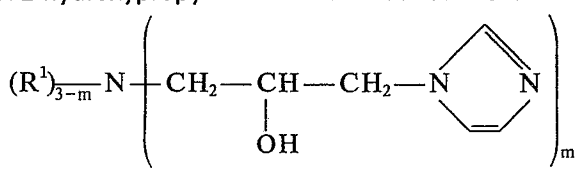

(II)

where R¹ is alkyl or cycloalkyl of 6 to 18 carbon atoms and m is 1 or 2, as corrosion inhibitors in hydrocarbons.

**Revendications**

1. Utilisation de dérivés du 2-hydroxypropylimidazole de formule I

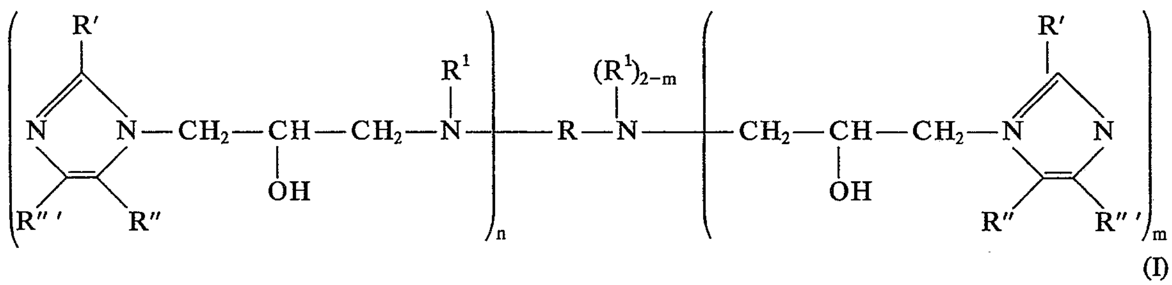

(I)

dans laquelle m représente les nombres 1 ou 2 et n les nombres 0 ou 1; R¹ représente un radical aliphatique ou cycloaliphatique à 6—21 atomes de carbone; R représente, dans le cas où n est égal à 0, un radical aliphatique, cycloaliphatique, aromatique ou araliphatiquw à 6—21 atomes de carbone et est de préférence semblable à R¹ ou, dans le cas où n est mis pour le nombre 1, il représente un radical aliphatique ou aromatique bivalent à 2—15 atomes de carbone ou le radical de formule

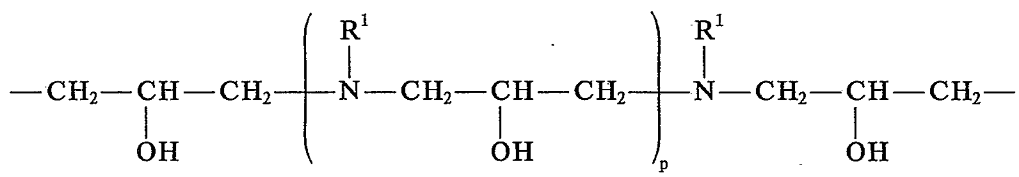

dans laquelle p est mis pour les nombres 0 ou 2; et R′, R″, R‴ sont mis pour des atomes d'hydrogène ou des radicaux alcoyle à 1—4 atomes de carbone, R″ ou R‴ pouvant en outre représenter un groupe nitro, en tant qu'inhibiteurs de corrosion dans des hydrocarbures.

2. Utilisation, en tant qu'inhibiteurs de corrosion dans des hydrocarbures, de dérivés du 2-hydroxypropylimidazole de formule

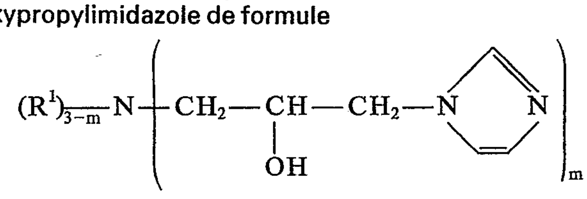

(II)

dans laquelle R¹ représente un radical alcoyle ou cycloalcoyle à 6—18 atomes de carbone et m représente les nombres 1 ou 2.